# EUROPEAN PATENT APPLICATION

(11) **EP 4 840 472 A1**
(43) Date of publication of application: **18.11.2026**
(21) Application number: 25175798.5
(22) Date of filing: 12.05.2025
(51) Int. Cl.: A61K 48/00, C12N 15/86

(54) **ASEPTIC MANUFACTURING OF LENTIVIRAL VECTORS**

(71) Applicant: AGC Biologics S.p.A., 20091 Bresso (MI) (IT)
(72) Inventor: CRIPPA, Luca, 20091 BRESSO (MI) (IT); CORBETTA, Samuele, 20091 BRESSO (MI) (IT); NERI, Margherita, 20091 BRESSO (MI) (IT); VALLANTI, Giuliana, 20091 BRESSO (MI) (IT)
(74) Representative: Studio Torta S.p.A.

(57) **Abstract**

The present invention relates to a method for manufacturing lentiviral vectors by aseptic processing comprising producing lentiviral vectors in a closed sterile cell culture unit; harvesting cell culture supernatant containing said lentiviral vectors into a first sterile container; clarifying said supernatant through sterile filters; collecting clarified supernatant in a second sterile container; purifying said lentiviral vectors using anion exchange chromatography in an automated system comprising single-use sterile components; formulating said lentiviral vectors by diafiltration using an automated system comprising single-use sterile components; and wherein all fluid transfers operated between steps and in each step are conducted through sterile connections to maintain aseptic conditions throughout the manufacturing process. It is further provided a system for manufacturing lentiviral vectors by aseptic processing comprising: a closed sterile cell culture unit configured to produce lentiviral vectors; a first sterile collection container fluidically connected to the cell culture unit via sterile tubing for harvesting cell culture supernatant; a clarification module comprising a series of sterile filters arranged in decreasing pore size order to obtain a concentrated lentiviral vector preparation, fluidically connected to the sterile collection container via sterile tubing and sterile connections; a second sterile collection container fluidically connected to the clarification module; an automated anion exchange chromatography unit comprising single-use sterile components configured for purifying the lentiviral vectors; and at least one automated tangential flow filtration unit comprising single-use sterile components configured for performing optional ultrafiltration to concentrate lentiviral vector preparation and diafiltration to formulate the lentiviral vectors.

## Description

### FIELD OF THE INVENTION

The present invention relates to methods for manufacturing lentiviral vectors under aseptic conditions. More specifically, the invention provides an improved manufacturing process for lentiviral vectors that maintains sterility throughout all processing steps without terminal sterilization filtration and a manufacturing system related thereto.

### BACKGROUND OF THE INVENTION

Lentiviral vectors (LVVs) are essential tools in gene therapy applications due to their ability to efficiently deliver genetic material into both dividing and non-dividing cells. They are commonly used for ex vivo gene therapy approaches and also for in vivo gene therapy applications. Several commercially approved gene therapies utilize lentiviral vectors (LVV) for gene delivery. Notable examples include Kymriah and Yescarta for blood cancers and Zynteglo for beta-thalassemia, Aucatzyl for r/r B-ALL, Lenmeldy^{™} for metachromatic leukodystrophy (MLD). In addition, many other clinical candidates are under investigation. Growing interest in the use of LVVs has created a strong demand for large volumes of vectors for preclinical, toxicology and clinical studies as well as ultimately for manufacturing of drug products approved for the market.

The manufacturing of clinical-grade lentiviral vectors presents significant challenges regarding sterility, yield, and potency. To manufacture lentiviral vectors (LVVs), cells such as human embryonic kidney cells (e.g., HEK 293T) are introduced with 3-4 plasmids through transfection. These plasmids typically include a transfer plasmid containing the gene of interest, along with packaging plasmids carrying essential viral proteins required for gene integration and self-assembly and the plasmid carrying the envelope protein, such as vesicular stomatitis virus G protein (VSV-G). The plasmids can be transiently transfected into the cells or utilized to establish a stable producer cell line that continuously expresses all the necessary genes for LVV production. As some of the gene products have a toxic effect inducible promoters (e.g., tet-off or tet-on) may be used in stable producer cell lines. By culturing transiently transfected cells or stable producer cells at suitable conditions LVV are released into the cell culture supernatant through the so called budding process. Purified lentiviral vectors are obtained by harvesting of the supernatant that is then subjected to several steps of clarification, purifications by capturing and polishing chromatography, concentration and formulation.

Conventional manufacturing processes for lentiviral vectors typically involve multiple open processing steps that increase the risk of contamination. To ensure sterility, terminal sterilizing filtration is commonly employed. However, this approach can result in significant loss of viral particles, with reported losses ranging from 30% to over 70% of functional vector particles during terminal filtration steps. Such losses substantially impact manufacturing efficiency and increase production costs.

Additionally, traditional open processing methods require specialized cleanroom facilities with extensive environmental monitoring, increasing manufacturing complexity and costs.

There remains a need in the art for improved methods for manufacturing lentiviral vectors that maintain aseptic conditions throughout the process, eliminate the need for terminal sterilization filtration, and provide high-quality vectors suitable for clinical applications.

### SUMMARY OF THE INVENTION

The present invention addresses the limitations of conventional manufacturing processes by providing a method for manufacturing lentiviral vectors through a complete aseptic processing approach. The method utilizes closed systems and sterile connections throughout the entire manufacturing process, eliminating the need for terminal sterile filtration while maintaining product sterility.

In one aspect, the invention provides a method for manufacturing lentiviral vectors comprising: producing lentiviral vectors in a closed sterile cell culture unit; harvesting cell culture supernatant containing said lentiviral vectors into a first sterile container; clarifying said supernatant through sterile filters; collecting clarified supernatant in a second sterile container; purifying said lentiviral vectors using anion exchange chromatography in an automated system comprising single-use sterile components; and formulating said lentiviral vectors by diafiltration using an automated system comprising single-use sterile components. Importantly, all fluid transfers operated between steps and in each step are conducted through sterile connections to maintain aseptic conditions throughout the manufacturing process.

In another aspect the present invention addresses a system for manufacturing lentiviral vectors by aseptic processing comprising: a closed sterile cell culture unit configured to produce lentiviral vectors; a first sterile collection container fluidically connected to the cell culture unit via sterile tubing for harvesting cell culture supernatant; a clarification module comprising a series of sterile filters arranged in decreasing pore size order fluidically connected to the sterile collection container via sterile tubing and sterile connections, a second sterile collection container fluidically connected to the clarification module; an automated anion exchange chromatography unit comprising single-use sterile components configured for purifying the lentiviral vectors; an automated tangential flow filtration unit comprising single-use sterile components configured for performing ultrafiltration to concentrate lentiviral vector preparation and/or diafiltration to formulate the lentiviral vectors; a sterile connection network connecting all components of the system to maintain aseptic conditions throughout the manufacturing process; and a control system configured to monitor and regulate the operation of the system components.

A key advantage of the invention is that it does not require a terminal sterilizing filtration step, which typically results in significant loss of viral particles. Instead, sterility is maintained throughout the entire process through the use of closed systems and sterile connections.

### DETAILED DESCRIPTION OF THE INVENTION

### Definitions

For the purpose of clarity and to facilitate understanding of the invention, the following definitions are provided for terms used throughout this specification.

These definitions are intended to provide context for interpreting the claims and should not be construed as limiting the scope of the invention.

"Aseptic processing" refers to a manufacturing technique performed in a manner that prevents contamination by microorganisms. It involves the handling of sterile materials in such a way that sterility is maintained throughout the process. In the context of this invention, aseptic processing means that all manufacturing steps are conducted using sterile equipment, components, and connections, without introducing environmental contaminants.

"Automated system" refers to equipment that performs operations with minimal manual intervention, controlled by programmed instructions. In the context of this invention, this includes chromatography systems such as ÄKTA^{™} systems from Cytiva, BioProcess^{™} systems from Sartorius, or BioSC^{™} systems from Novasep. It also encompasses automated filtration systems for tangential flow filtration (TFF) such as Cogent^{®} M TFF systems from Millipore Sigma, KrosFlo^{®} KR2i or KMPi TFF systems from Repligen, Cadence^{™} Automated TFF systems from Pall, or ÄKTA^{™} flux S/P systems from Cytiva.

"Cell culture supernatant" refers to the liquid medium surrounding cells in a culture system, which contains secreted products such as lentiviral vectors, proteins, and other biomolecules. According to the present invention the supernatant is harvested after the cells have been cultured under conditions suitable for lentiviral vector production.

"Clarification filters" refers to filtration devices used to remove cells, cell debris, and other particulates from cell culture harvests. Commercial examples include but are not limited to Millistak+^{®} pods from Millipore Sigma, Supracap^{™} capsules from Pall, Sartoclear^{®} filters from Sartorius, or POD^{®} filters from Parker, available in various pore sizes typically ranging from 0.45 µm to 1.5 µm.

"Closed sterile cell culture device" or "Closed sterile cell culture unit" refers to a bioreactor or similar vessel designed to maintain a sterile environment for cell culture without exposure to the external environment during operation. Examples include, but are not limited to, fixed-bed bioreactors, stirred-tank bioreactors, wave bioreactors, hollow fiber bioreactors, packed-bed bioreactors (such as iCELLis^{®} systems from Pall Corporation), perfusion bioreactors, single-use bioreactor bags (such as WAVE^{™} bioreactors from Cytiva), microcarrier culture systems, and combinations thereof.

"Fluid": refers to any liquid or gas that can flow and is used in the context of the manufacturing process. In this invention, fluid specifically refers to liquids including cell culture media, buffers, solutions, clarified supernatant, and formulated lentiviral vector solutions that are transferred between the several components used to perform the method according to the present invention.

"Diafiltration" refers to a membrane-based separation technique that uses buffer exchange to replace the original solution with a different buffer while retaining the product of interest. It uses the same equipment as ultrafiltration but involves the addition of new buffer as filtrate is removed. Commercial options include Cogent^{®} TFF systems from Millipore Sigma, ÄKTA^{™} flux systems from Cytiva, or KrosFlo^{®} systems from Repligen, with single-use kits such as Pellicon^{®} cassettes (Millipore Sigma), Hollow Fiber cartridges (Repligen), or ReadyToProcess^{™} filters (Cytiva).

"Single-use sterile components" refers to pre-sterilized disposable parts that are used once and then discarded, eliminating the need for cleaning and re-sterilization. These include but are not limited to bags, tubing, connectors, sensors, filters, and chromatography devices designed for one-time use in biopharmaceutical manufacturing.

"Sterile" refers to the state of being free from viable microorganisms, including bacteria, fungi, viruses, and spores. In the context of this invention, components, containers, and systems described as "sterile" have been subjected to validated sterilization processes to ensure the absence of viable microorganisms. Sterilization methods can be broadly categorized as heat, chemical, radiation, and filtration.

"Sterile connection" refers to a means of connecting two sterile fluid pathways while maintaining the sterility of both. Sterile connections are specialized systems or devices designed to enable the transfer of materials (such as liquids, gases, or solids) between two environments while maintaining sterility and preventing contamination. This may include aseptic connectors (such as Pall's Kleenpak^{™} connectors or Cytiva's ReadyMate^{™} connectors), sterile tubing welders, or other validated aseptic connection technologies. Sterile connections are essential in industries and applications where maintaining a sterile environment is critical, such as medicine, pharmaceuticals, and biotechnology. Key features of sterile connections include: a) sterility assurance they are engineered to ensure complete sterility during the transfer process, preventing the introduction of microorganisms or unwanted particles, b) durable materials they are made from materials that can withstand sterilization methods like autoclaving, gamma irradiation, or chemical sterilization, c) ease of use they are designed for easy connection and disconnection without compromising sterility, d) they can be single use or reusable depending on the application, they can be disposable to minimize contamination risks or reusable with proper sterilization protocols. There are several types of sterile connections: 1) sterile quick connectors that allow fast and secure connections between tubing or systems without contamination, 2) aseptic transfer systems used for transferring liquids or materials between sterile containers, 3) single-use connections often preferred for critical applications to reduce contamination risks and simplify processes.

"Ultrafiltration" refers to a separation process using membranes with specific molecular weight cut-offs to concentrate solutions by removing water and small solutes while retaining larger molecules like viral vectors. Commercial systems include Cogent^{®} TFF systems from Millipore Sigma, ÄKTA^{™} flux from Cytiva, or KrosFlo^{®} systems from Repligen, with single-use kits such as Pellicon^{®} cassettes (Millipore Sigma), Hollow Fiber cartridges (Repligen), or ReadyToProcess^{™} filters (Cytiva).

### Method for Manufacturing Lentiviral Vectors

The present invention provides a comprehensive method for the aseptic manufacturing of lentiviral vectors suitable for clinical applications. Each aspect of the invention will now be described in detail.

### Lentiviral vectors (LVVs)

The present invention relates to the manufacturing of LVVs. LVVs are gene delivery vehicles derived from lentiviruses. A detailed list of lentiviruses may be found in Coffin et al ("Retroviruses" 1997 Cold Spring Harbour Laboratory Press Eds: JM Coffin, SM Hughes, HE Varmus pp 758-763). In brief, lentiviruses can be divided into primate and non-primate groups. Examples of primate lentiviruses include, but are not limited to: the human immunodeficiency virus (HIV), the causative agent of human acquired-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

In a preferred embodiment, the lentiviral vector is derived from an HIV lentivirus, more preferably the lentiviral vector is derived from an HIV-1 lentivirus.

An exemplary lentiviral vector to be used in the method of the invention comprises at least the following portion of lentiviral genome: a) a 5' long terminal repeat (LTR); b) the packaging sequence psi; c) a Rev Response Element (RRE); d) a promoter operably linked to a gene of interest; e) a 3' long terminal repeat (LTR). In a preferred embodiment the U3 region of the 5' LTR is replaced with a heterologous promoter selected from the group consisting of: a cytomegalovirus (CMV) promoter, a Rous Sarcoma Virus (RSV) promoter, or a Simian Virus 40 (SV40); thus rendering lentiviral transcription tat independent. In a further preferred embodiment, the 3' LTR sequence contains a deletion of the U3 region (i.e. the vector is a self-inactivating vector or SIN vector). The lentiviral vector may further comprise a lentiviral central poly-purine tract (cPPT) and Woodchuck Hepatitis Virus (WHP) Posttranscriptional Regulatory Element (WPRE)).

### Cells

Lentiviral vectors can be suitably propagated in cells (sometimes referred to as "host cells'). All cell lines suitable for the manufacturing of viral vectors can be used in the present invention. In one embodiment the packaging cell line to be used in the present invention is selected from HEK293, HEK293 T, HEK 293E, HEK 293FT, 293 Vec, TE671, HT1080 or HeLa cell line. In a preferred embodiment the packaging cell line is HEK 293 T.

### Production

The first step in the method involves producing lentiviral vectors in a closed sterile cell culture unit. The closed sterile cell culture unit may be selected from the group consisting of a fixed-bed bioreactor, a stirred-tank bioreactor, a wave bioreactor, a hollow fiber bioreactor, a packed-bed bioreactor, a perfusion bioreactor, a single-use bioreactor bag, a microcarrier culture system, and combinations thereof. In a preferred embodiment, the closed sterile cell culture unit is a single-use packed-bed bioreactor.

The production of lentiviral vectors in the closed sterile cell culture unit can be accomplished through several approaches.

According to one embodiment LVVs are produced in the cell culture device by plasmid DNA transient transfection of cells i.e. in the method of the invention at least two expression plasmids carrying two different genes are transiently co-transfected into a cell line (also called packaging cell line). According to a preferred aspect of the invention, the cells are co-transfected at least with: a packaging plasmid carrying lentiviral Gag/Pol genes, a plasmid carrying the gene encoding the envelope protein of interest and the transfer plasmid carrying the essential lentiviral genome elements, as disclosed above, and the gene of interest. In another preferred embodiment the lentiviral regulatory protein Rev may be further expressed in trans on a fourth separate plasmid. In a further aspect of the invention lentiviral protein tat may be further expressed on a fifth separate plasmid. Examples of suitable env genes include, but are not limited to, VSV-G env, MLV 4070 env, RD114 env, RD114-TR, RD114pro, baculovirus 5 GP64 env, GALV or envelope proteins derived from Measle virus. In a preferred embodiment, in the method of the invention the multi-plasmid DNA transfection is performed using a third generation LVVs viral vector system composed of four plasmids i.e.: one plasmid carrying the lentiviral gag/pol genes, one plasmid carrying lentiviral rev gene, one plasmid carrying the gene encoding the envelope protein, preferably the gene encoding the VSV-G envelope protein, and one plasmid carrying the transfer vector including the required portion of the lentiviral genome and the foreign gene of interest. In a preferred embodiment the method of the invention comprises inoculating cells into the closed sterile cell culture unit; culturing said cells for expansion for at least one day; transfecting the cells; and cultivating the cells for at least 24 hours after transfection. All steps are performed using sterile containers and sterile connections to operate transfer of fluids to maintain aseptic conditions.

In another embodiment lentiviral vectors can be produced by inoculating into the closed sterile cell culture unit stable packaging cells capable of constitutively expressing lentiviral gag/pol, rev and VSV-G env for expansion culturing such stable packaging cells for at least one day in the sterile cell culture device; transfecting the cells with the transfer vector plasmid only; and cultivating the cells for at least 24 hours after transfection.

In another embodiment, lentiviral vectors can be produced by culturing a stable producer cell line capable of constitutively expressing all lentiviral vector components and the gene of interest in said closed sterile cell culture unit under conditions suitable for lentiviral vector production.

According to the present invention the inoculum of the cells and transfection of the cells are performed in a sterile manner using sterile components and sterile connections for the transfer of fluids required to perform such steps of the method.

The skilled man may use any of the cell cultivation operating conditions known in the art and applicable to the closed sterile cell culture unit in use for supplying the cell culture medium containing nutrients to the cells. For example, the iCELLis^{®} Single-Use Fixed-Bed Bioreactor Systems allows to operate cell culturing in three different modes: 1) "in batch mode" according to which the cells are cultivated using a constant volume of cell culture medium into the bioreactor for the required period of time up to the media consumption (i.e.: the exhaustion of the nutrients required for cell growth and the accumulation of cell metabolites) and, upon reaching medium consumption, it is necessary to drain completely the exhausted medium from the bioreactor and fill it with the fresh medium 2) "in perfusion mode" according to which during the cell culture, the fresh medium is added into the bioreactor at a constant flow rate and the exhausted medium is removed at the same flow rate to be wasted or harvested, while maintaining a constant total volume of cell culture medium into the bioreactor and allowing a constant supply of nutrients to the cell culture and a constant removal of cell metabolites; 3) "re-circulation mode" according to which the cells are cultivated in a constant amount of cell culture medium into the bioreactor, which is connected with an external container serving as reservoir of medium, from which additional culture medium is added at a constant flow rate into the bioreactor (as in perfusion mode) and removed at the same flow rate to be carried back to the reservoir. The culture in re-circulation mode allows an increase of the total volume of medium used for the cell culture. Each of the above-mentioned operational modes or a combination thereof may be employed in the method of the invention to culture the cells for expansion.

### Harvesting and Clarification

Following the production phase, the cell culture supernatant containing the lentiviral vectors is harvested into a first sterile container. The harvested supernatant is then subjected to a clarification step to remove cellular debris and large particulates.

The clarification step comprises sequentially passing the cell culture supernatant through at least two sterile filters of decreasing pore size. All sterilizable filters available on the market can be used in the method of the invention. This sequential filtration approach ensures efficient removal of cellular debris while minimizing loss of viral particles. In one embodiment the maximum pore size of the filters employed in the invention is 1.5µm. In another embodiment the minimum pore size of the filters is 0.45µm. In a preferred embodiment, the clarification step comprises sequentially passing the cell culture supernatant through at least one sterile filters having pore size of 1.5µm and at least one filter having 0.45µm.

The clarified supernatant is collected in a second sterile container through a sterile connection.

### Concentration

Optionally, in some embodiments, before anion exchange chromatography, the clarified supernatant may be concentrated, to obtain a concentrated lentiviral vector preparation, by ultrafiltration using an automated tangential flow filtration system comprising single-use sterile components. Several examples of automated systems to operate tangential flow filtration are available on the market and may be used to practice the present invention including Cogent^{®} M TFF systems from Millipore Sigma, KrosFlo^{®} KR2i or KMPi TFF systems from Repligen, Cadence^{™} Automated TFF systems from Pall, or ÄKTA^{™} Readyflux and flux S/P systems from Cytiva, which can be used for concentration and diafiltration operations in an aseptic manner using single-use flow paths and filters.

In the method of the present invention, ultrafiltration can be performed using sterile single use ultrafiltration cartridges and hollow fiber systems. In a preferred embodiment, ultrafiltration is performed using sterile single use hollow fiber filters. More preferably the sterile single use hollow fiber filter has a molecular weight cut-off between 100 kDa and 750 kDa. The concentrated clarified supernatant is collected in a sterile container. This concentration step helps to reduce the volume of material to be processed in subsequent purification steps and at the same time reduces the amount of contaminating molecules smaller than the hollow-fiber pore-size

### Purification

According to the method of the invention, lentiviral vectors are purified by anion exchange chromatography in an automated system comprising single-use sterile components. Several examples of automated system to operate chromatography are available on the market and may be used to practice the present invention including ÄKTA^{™} systems from Cytiva, BioProcess^{™} systems from Sartorius, or BioSC^{™} systems from Novasep, which can be configured with single-use flow paths for aseptic operation. In a preferred embodiment the purification step is operated with a sterile single-use membrane-based anion exchange chromatography unit. In a preferred embodiment, the chromatographic membrane is functionalized with a quaternary amine. This purification step allows for the selective capture of lentiviral vectors while removing process-related impurities.

### Post-Purification Concentration

In some embodiments, optionally the method further comprises concentrating the lentiviral vectors by ultrafiltration using an automated system comprising single-use sterile components after purification and before formulation.

### Formulation

The final step in the method involves formulating the lentiviral vectors by diafiltration using an automated system comprising single-use sterile components. The formulation step is operated with a single-use sterile hollow fiber filter. This step allows for buffer exchange to place the lentiviral vectors in an appropriate final formulation buffer suitable for the intended application.

### Maintaining Aseptic Conditions

A key aspect of the invention is that all components used for the process are sterile, and those components in direct contact with the cells or the lentiviral vectors are in a preferred configuration of the invention single use. In addition, throughout the method of the invention, fluid transfers operated between steps and during each step are conducted through sterile connections to maintain aseptic conditions throughout the manufacturing process. This eliminates the need for terminal sterilization filtration, which can result in significant loss of viral particles.

### System

According to another aspect of the present invention, it is provided a system for manufacturing lentiviral vectors by aseptic processing comprising:
1. A closed sterile cell culture unit configured to produce lentiviral vectors;
2. A first sterile collection container fluidically connected to the cell culture unit via sterile tubing for harvesting cell culture supernatant;
3. A clarification module comprising a series of sterile filters arranged in decreasing pore size order fluidically connected to the sterile collection container via sterile tubing and sterile connections;
4. A second sterile collection container fluidically connected to the clarification module;
5. An automated anion exchange chromatography unit comprising single-use sterile components configured for purifying the lentiviral vectors;
6. At least one automated tangential flow filtration unit comprising single-use sterile components configured for performing ultrafiltration to concentrate lentiviral vector preparation and/or diafiltration to formulate the lentiviral vectors;
7. A sterile connection network connecting all components of the system to maintain aseptic conditions throughout the manufacturing process; and
8. A control system configured to monitor and regulate the operation of the system components.

In a preferred embodiment of the system according to the present invention, the closed sterile cell culture unit of the invention is a bioreactor selected from the group consisting of a fixed-bed bioreactor, a stirred-tank bioreactor, a wave bioreactor, a hollow fiber bioreactor, a packed-bed bioreactor, a perfusion bioreactor, a single-use bioreactor bag, a microcarrier culture system, and combinations thereof. In a preferred embodiment of the system according to the invention, the closed sterile cell culture unit is a single-use packed-bed bioreactor. Such bioreactor is configured to maintain sterility throughout the cell culture period including means for sterile addition and collection of fluids configured to perform manufacturing of lentiviral vectors such as cell inoculation, cell culture medium, supply of reagents including transfection reagents and sterile sampling, a temperature control system, a pH monitoring system and a dissolved oxygen monitoring system. In a preferred embodiment of the invention, the bioreactor is configured to be operated to perform multiple types of manufacturing processes including batch, fed-batch, perfusion, fill and draw, or recirculation processes or a combination thereof.

In a preferred embodiment, the clarification module of the system comprises at least two sterile filters arranged in sequence of decreasing pore size. In one preferred embodiment, such filters have maximum pore size of 1.5 µm. In another preferred embodiment such filters have minimum pore size of 0.45 µm. In a particularly preferred embodiment, the clarification module of the system comprises at least two sterile filters arranged in sequence of decreasing pore size wherein the maximum pore size is 1.5 µm and the minimum pore size is 0.45 µm.

In one embodiment the anion exchange chromatography unit of the system comprises a sterile single-use membrane-based anion exchange chromatography device, an automated buffer management system for loading, washing, and elution operations, single-use sterile tubing, sterile kits and sterile connections for transfer of fluids configurated to maintain sterility throughout the purification process. In a particularly preferred embodiment, the single-use membrane-based anion exchange chromatography unit comprises chromatographic membrane functionalized with a quaternary amine.

In one embodiment the tangential flow filtration unit of the system comprises an automated buffer management system for performing ultrafiltration and/or diafiltration, a single-use sterile device selected from an ultrafiltration cartridge or an hollow fiber filter, a single-use sterile tubing, sterile kits and sterile connections for transfer of fluids configurated to maintain sterility throughout the ultrafiltration process and/or diafiltration process. More preferably, the tangential flow filtration unit of the system comprises a single use sterile hollow fiber filter.

In one preferred embodiment the system of the invention comprises one or more integrated control systems configured to monitor and control all process parameters, perform automated fluid transfers between system components, maintain records of process parameters for quality assurance, alert operators of any deviations from set parameters; and control maintenance of aseptic conditions throughout the process.

The method of the present invention offers several advantages over conventional manufacturing processes for lentiviral vectors:
1. Elimination of terminal sterilization filtration, resulting in higher yields of functional viral particles
2. Reduced risk of contamination through the use of closed systems and sterile connections
3. Increased process scalability through the use of automated systems and single-use components
4. Improved reproducibility through standardized processing
5. Reduced dependency on specialized cleanroom facilities
6. Lower process development and validation costs
7. Greater flexibility and faster turnaround times for manufacturing

### EXAMPLES

### Example 1: Production of Lentiviral Vectors Using Transient Transfection

HEK293T cells were inoculated into a single-use packed-bed bioreactor iCellis Nano at a density of 5000cells/cm2. The cells were cultured at 37°C and 5% CO2 for for three days. Transfection was performed using a mixture of four plasmids (transfer vector, packaging plasmid, Rev-expressing plasmid, and envelope plasmid) complexed with polyethylenimine (PEI). The transfection mixture was added to the bioreactor through a sterile connection.

The cells were cultured for an additional 39 hours post-transfection. The cell culture supernatant was harvested into a sterile container using a sterile peristaltic pump and sterile tubing assembly. The harvested supernatant was clarified by sequential filtration through a 1.5µm filter followed by a 0.45µm filter using sterile connections between each filtration step.

Supernatant was purified through a chromatographic purification step using a capsule containing a sterile Cytiva Mustang Q membrane (strong ion exchange - quaternary amine) connected to an AKTA ready system through a sterile, single use flow path. The clarified supernatant was applied onto the chromatographic membrane through single-use sterile flow paths and connections, taking advantage of an automated method. After loading was completed, elution of the lentiviral vectors bound to the resin was obtained with NaCl containing D-PBS buffers. The eluted lentiviral vector was immediately diluted with D-PBS to reduce salt concentration. Around 50-60% of the applied lentiviral vectors was recovered in the eluted peak, in terms of infectious viral titre, a performance similar or better than standard nonsterile resins. Residual host cell proteins and total DNA removal was similarly comparable or better than what obtained with standard non-sterile resin.

The purified lentiviral vectors were then subjected to a following step for further concentration and formulated by diafiltration into a final formulation buffer. This was obtained using a 750 kDa sterile hollow fiber, connected via sterile flow path and aseptic connectors to an automated TFF system with single use flow path. After a concentration phase, final formulation medium was progressively added through sterile connections to execute the diafiltration step and formulate the lentiviral vectors.

As all the purification and concentration steps were executed in a close system, with sterile connectors and with sterile components, there was no need to execute any final sterilizing filtration. As the filtration step has a significative impact on overall LVV recovery, with losses up to 70-80%, the possibility to avoid this step resulted in an overall much higher process recovery.

## Claims

1. A method for manufacturing lentiviral vectors by aseptic processing comprising:
a. producing lentiviral vectors in a closed sterile cell culture unit;
b. harvesting cell culture supernatant containing said lentiviral vectors into a first sterile container;
c. clarifying said supernatant through sterile filters;
d. collecting clarified supernatant in a second sterile container;
e. purifying said lentiviral vectors using anion exchange chromatography in an automated system comprising single-use sterile components;
f. formulating said lentiviral vectors by diafiltration using an automated system comprising single-use sterile components; and
wherein all fluid transfers operated between steps and in each step are conducted through sterile connections to maintain aseptic conditions throughout the manufacturing process.

2. The method according to claim 1 wherein the method does not comprise a step of sterilizing filtration.

3. The method according to claim 1 or 2, further comprising concentrating said lentiviral vectors by ultrafiltration using an automated system comprising single-use sterile components after step e and before step f.

4. The method according to anyone of claim 1 to 3 further comprising concentrating the clarified supernatant by ultrafiltration using an automated tangential flow filtration system comprising single-use sterile components after step d and before step e, wherein the concentrated clarified supernatant is collected in a further sterile container.

5. The method according to claims 1 to 4, wherein producing lentiviral vectors in the closed sterile cell culture device comprises:
a. inoculating cells into the closed sterile cell culture device;
b. culturing said cells for expansion for at least one day;
c. transfecting said cells; and
d. cultivating the cells for at least 24 hours after transfection
wherein all steps from a to d are performed using sterile containers and sterile connections to operate transfer of fluids to maintain aseptic conditions.

6. The method according to claims 1 to 5 wherein producing lentiviral vectors comprises culturing a stable producer cell line capable of constitutively expressing lentiviral vector components in said closed sterile cell culture device under conditions suitable for lentiviral vector production

7. The method according to any preceding claims wherein the clarification step comprises sequentially passing the cell culture supernatant through at least two sterile filters of decreasing pore size to remove cellular debris and large particulates from viral vector preparation.

8. The method according to claim 7 wherein the minimum pore size of the filter is 0.45 mm.

9. The method according to claim 7 wherein the maximum pore size of the filter is 1.5 mm

10. The method according to any preceding claims wherein the purification step is operated with a sterile single-use membrane-based anion exchange chromatography unit comprising a chromatographic membrane.

11. The method according to claim 10 wherein the chromatographic membrane is functionalized with a quaternary amine.

12. The method according to any of preceding claims wherein the formulation step is operated with a single use sterile hollow fiber filter.

13. The method according to anyone of claims from 3 to 12 wherein the concentration step is operated with a single use sterile hollow fiber filter.

14. A system for manufacturing lentiviral vectors by aseptic processing comprising:
- a closed sterile cell culture unit configured to produce lentiviral vectors;
- a first sterile collection container fluidically connected to the cell culture unit via sterile tubing for harvesting cell culture supernatant;
- a clarification module comprising a series of sterile filters arranged in decreasing pore size order to obtain a concentrated lentiviral vector preparation, fluidically connected to the sterile collection container via sterile tubing and sterile connections;
- a second sterile collection container fluidically connected to the clarification module;
- an automated anion exchange chromatography unit comprising single-use sterile components configured for purifying the lentiviral vectors
- at least one automated tangential flow filtration unit comprising single-use sterile components configured for performing optional ultrafiltration to concentrate lentiviral vector preparation and diafiltration to formulate the lentiviral vectors.

15. The system according to claim 14 wherein the closed sterile cell culture unit is selected from the group consisting of a fixed-bed bioreactor, a stirred-tank bioreactor, a wave bioreactor, a hollow fiber bioreactor, a packed-bed bioreactor, a perfusion bioreactor, a single-use bioreactor bag, a microcarrier culture system, and combinations thereof.

16. The system according to claim 5, wherein the closed sterile cell culture unit is a single use packed-bed bioreactor.
